# DEMANDE DE BREVET EUROPEEN

(11) **EP 1 941 930 A1**
(43) Date de publication de la demande: **09.07.2008**
(21) Numéro de dépôt: 07123805.9
(22) Date de dépôt: 20.12.2007
(51) Int. Cl.: A61Q 5/06, A61Q 5/12, A61K 8/895, A61K 8/89

(54) **Composition comprenant un composé silicone et un organosilane particulier**

(30) Priorité: 20.12.2006 FR 0655753
(71) Demandeur: L'Oréal, 75008 Paris (FR)
(72) Inventeur: BENABDILLAH, Katarina, 95130, LE PLESSIS-BOUCHARD (FR)
(74) Mandataire: Fevrier, Murielle Françoise E.

(57) **Abrégé**

La présente invention a pour objet une composition pour le traitement des fibres kératiniques, et en particulier des fibres kératiniques humaines telles que les cheveux.
La présente invention a pour objet une composition pour la coloration des fibres kératiniques comprenant :
- au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- au moins un alcoxysilane comprenant un seul atome de silicium.

La composition de l'invention permet d'obtenir des gainages particulièrement rémanents, notamment aux lavages répétés.

## Description

La présente invention a pour objet une composition pour le traitement des fibres kératiniques, et en particulier des fibres kératiniques humaines telles que les cheveux.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Ainsi, pour remédier à cela, il est maintenant usuel d'utiliser des produits de coiffage qui permettent de conditionner les cheveux en leur apportant notamment du corps, de la masse, de la brillance ou du volume. Ces produits de coiffage sont généralement des compositions cosmétiques capillaires comprenant un ou plusieurs polymères qui présentent une affinité pour les cheveux et qui ont le plus souvent pour fonction de former un film à leur surface en vue de modifier leurs propriétés superficielles, notamment pour les conditionner. Pour obtenir un tel effet, il est connu en particulier d'utiliser des polysiloxanes, notamment ceux décrits dans les documents FR 2 799 955, FR 2 799 956, FR 2 799 970 et FR 2 799 971.

Un inconvénient lié à l'utilisation de ces compositions capillaires réside dans le fait que les effets cosmétiques conférés par de telles compositions ont tendance à disparaître, notamment dès le premier shampooing.

Afin de remédier à cet inconvénient, il est envisageable d'accroître la rémanence du dépôt de polymères en effectuant directement une polymérisation de certains monomères au niveau des cheveux. Le document US 4 344 763 décrit une composition de fixation des cheveux à partir d'une silicone réactive aminoalkylalcoxysilane et un titanate d'ester. Il est aussi connu d'effectuer des gainages des cheveux à partir d'une composition comprenant un monomère électrophile de type cyanoacrylate, notamment dans les demandes de brevet FR 2 833 489. Une telle composition permet d'obtenir des cheveux parfaitement gainés et non gras. Cependant, le gainage obtenu ne donne pas une totale satisfaction face aux agents extérieurs tels que le lavage et la transpiration. Par ailleurs le gainage obtenu est sensible aux corps gras tels que le sébum.

Les documents WO01/96450, GB 2407496 et EP 465 744 décrivent l'utilisation de silicones réactives particulières pour la réalisation de film sur la peau. Le document WO 01/96450 et GB 2 407 496 décrivent une formulation en une partie qui comprend un polysiloxane ayant des groupes terminaux trialkoxyalkylsilyle un catalyseur, un solvant et éventuellement un alkoxysilane et des charges. Ces compositions permettent d'obtenir par condensation un film sur la peau. Le document EP 465 744 décrit l'utilisation de polysiloxane à groupements aliphatiques insaturés pour réaliser des dispositifs médicaux à usage topique.

Le but de la présente invention est de développer un nouveau système de traitement des cheveux qui permet d'obtenir une fixation durable des cheveux tout en conservant de bonnes propriétés cosmétiques.

Ainsi, l'invention a pour objet une composition de traitement des fibres kératiniques comprenant au moins un composé X et au moins un composé Y, l'un au moins des composé X et Y étant un composé siliconé, les composés X et Y étant susceptibles de réagir ensemble par une réaction d'hydrosilylation, de condensation, ou de réticulation en présence d'un peroxyde, lorsqu'ils sont mis en contact l'un avec l'autre, et au moins un alcoxysilane comprenant un seul atome de silicium.

L'invention a aussi pour objet un procédé de traitement des cheveux qui consiste à appliquer sur les cheveux au moins un composé X et au moins un composé Y, l'un au moins des composé X et Y étant un composé siliconé, les composés X et Y étant susceptibles de réagir ensemble par une réaction d'hydrosilylation en présence d'un catalyseur, une réaction de condensation, ou une réaction de réticulation en présence d'un peroxyde, lorsqu'ils sont mis en contact l'un avec l'autre, et au moins un alcoxy silane comprenant un seul atome de silicium.

L'invention a aussi pour objet l'utilisation de l'association des composés X, Y et d'un alcoxysilane comprenant un seul atome de silicium pour le traitement des cheveux, notamment pour l'obtention d'un gainage résistant aux shampoings des cheveux.

Un autre objet de l'invention concerne un kit pour le traitement des fibres kératiniques comprenant au moins deux compositions conditionnées séparément, le kit comprenant au moins deux composés X et Y, aptes à réagir ensemble, l'un au moins de ces composés étant siliconé, et au moins un alcoxysilane comprenant un seul atome de silicium.

Le procédé de l'invention permet d'obtenir in situ un gainage rémanent qui est homogène, lisse et possède une excellente adhésion sur cheveux. Par ailleurs, on a constaté de façon surprenante que les cheveux pouvaient être mis en forme par la formation de méchés c'est-à-dire d'ensembles individualisés cohésifs d'au moins 10 cheveux, chacun collé entre eux résistant aux shampoings. De tels ensembles individualisés cohésifs permettent d'obtenir des coiffures sophistiquées, notamment des coiffures festives résistantes aux lavages.

### Composés X et Y

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO⁻ , -COO- , -OH , -NH₂ , -NH-,-NR-, -SO₃H, -SO₃⁻, -OCH₂CH₂-, -O-CH₂CH₂CH₂-, -O-CH₂CH(CH₃)-, -NR₃⁺, -SH, - NO₂, I, Cl, Br, -CN, -PO₄ ³⁻, -CONH- , -CONR-, -CONH₂, -CSNH-, -SO₂- , -SO-, - SO₂NH-, -NHCO- , -NHSO₂- , -NHCOO-, -OCONH-, -NHCSO- et -OCSNH-, R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Les composés X et Y peuvent réagir ensemble à une température variant entre la température ambiante et 180 °C. Avantageusement, les composés X et Y sont susceptibles de réagir ensemble à température ambiante (20 ± 5 °C) et pression atmosphérique, avantageusement en présence d'un catalyseur, par une réaction d'hydrosilylation ou une réaction de condensation, ou une réaction de réticulation en présence d'un peroxyde.

### 1- Composés X et Y susceptibles de réagir par hydrosilylation

Selon un mode de réalisation, les composés X et Y sont susceptibles de réagir ensemble par hydrosilylation, cette réaction pouvant être de manière simplifiée schématisée comme suit : 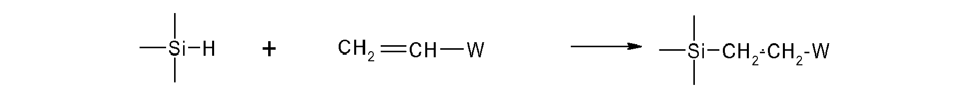 avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatiques insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule : 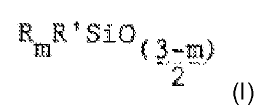 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle ;
- m est égal à 1 ou 2 ; et
- R' représente :
   - un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
   - un groupement hydrocarboné cyclique insaturé comprenant de 5 à 8 atomes de carbone comme par exemple un groupe cyclohexényle.

De préférence R' est un groupement hydrocarboné aliphatique insaturé, de préférence un groupe vinyle.

Selon un mode de réalisation particulier, le polyorganosiloxane comprend également des unités de formule : 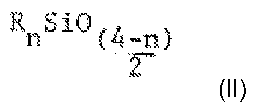 dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé B par hydrosilylation. On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de "MDTQ", la résine étant décrite en fonction des différentes unités monomériques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2}.

Dans les motifs M, D, T définis précédemment, au moins un des groupes méthyle peut être substitué par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle SiO_{4/2} dans laquelle l'atome de silicium est lié à quatre atomes d'hydrogène eux mêmes liés au reste du polymère. Comme exemples de telles résine, on peut citer les résines de silicone MT telles que les poly(phenyl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les organosiloxanes comprenant au moins une unité alkylhydrogènosiloxane de formule suivante : 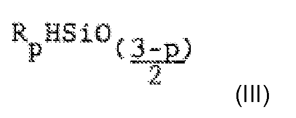 dans laquelle :
R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogènosiloxanes peuvent comprendre en outre des unités de formule : 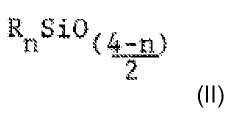 telles que définies plus haut.

Le composé Y peut être une résine de silicone comprenant au moins un motif choisis parmi les motifs M, D, T, Q tels que définis ci-dessus et comprenant au moins un groupe Si-H telles que les poly(methyl-hydridosilsesquioxane) commercialisées sous la référence SST-3MH1.1 par la société Gelest.

De préférence, ces composés Y organosiloxanes comprennent de 0,5 à 2,5 % en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule (CH₃)₃SiO_{1/2}.

Avantageusement, les organosiloxanes Y comprennent au moins deux unités alkylhydrogènosiloxane de formule (H₃C)(H)SiO et comprennent éventuellement des unités (H₃C)₂SiO.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique / silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et / ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :
a) les polyesters à insaturation(s) éthylénique(s) :
   II s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
      - d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.
b) les polyesters à groupes (méth)acrylate latéraux et / ou terminaux :
   Il s'agit d'un groupe de polymères de type polyester obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
      - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
   De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule).
c) les polyuréthannes et / ou polyurées à groupes (méth)acrylate, obtenus par polycondensation :
   - de diisocyanates, triisocyanates et / ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 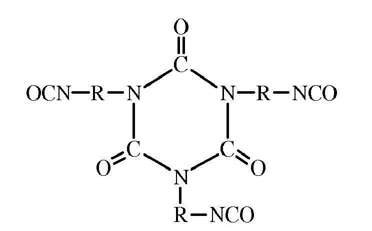 résultant de la trimérisation de 3 molécules de diisocyanates OCN-R-CNO, où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et / ou de polyamines, notamment de diamines, aliphatiques, cycloaliphatiques et / ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes / polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule, EBECRYL^{®} 230 : masse molaire 5000, 2 fonctions acrylate par molécule, EBECRYL^{®} 270 : masse molaire 1500, 2 fonctions acrylate par molécule, EBECRYL^{®} 8402 : masse molaire 1000, 2 fonctions acrylate par molécule, EBECRYL^{®} 8804 : masse molaire 1300, 2 fonctions acrylate par molécule, EBECRYL^{®} 220 : masse molaire 1000, 6 fonctions acrylate par molécule, EBECRYL^{®} 2220 : masse molaire 1200, 6 fonctions acrylate par molécule, EBECRYL^{®} 1290 : masse molaire 1000, 6 fonctions acrylate par molécule, EBECRYL^{®} 800 : masse molaire 800, 6 fonctions acrylate par molécule).
   On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁-C₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL^{®} 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL^{®} 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre :
   - au moins un diépoxyde choisi par exemple parmi :
      (i) l'éther diglycidylique de bisphénol A,
      (ii) une résine diépoxy résultant de la réaction entre l'éther diglycidylique de bisphénol A et l'épichlorhydrine,
      (iii) une résine époxyester à extrémités α,ω-diépoxy résultant de la condensation d'un diacide carboxylique ayant de 3 à 50 atomes de carbone avec un excès stoechiométrique de (i) et / ou (ii),
      (iv) une résine époxyéther à extrémités α,ω-diépoxy résultant de la condensation d'un diol ayant de 3 à 50 atomes de carbone avec un excès stoechiométrique de (i) et / ou (ii),
      (v) les huiles naturelles ou synthétiques portant au moins 2 groupes époxyde, telles que l'huile de soja époxydée, l'huile de lin époxydée et l'huile de vernonia époxydée,
      (vi) un polycondensat phénol-formaldéhyde (résine Novolac^{®}), dont les extrémités et/ou les groupes latéraux ont été époxydés,
         et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.
f) les poly(méth)acrylates d'(alkyle en C₁₋₅₀), ledit alkyle étant linéaire, ramifié ou cyclique, comportant au moins deux fonctions à double liaison éthylénique portées par les chaînes hydrocarbonées latérales et/ou terminales.
   De tels copolymères sont commercialisés par exemple sous les dénominations IRR^{®} 375, OTA^{®} 480 et EBECRYL^{®} 2047 par la société UCB.
g) les polyoléfines telles que le polybutène, le polyisobutylène,
h) les perfluoropolyéthers à groupes acrylate obtenus par estérification, par exemple par l'acide (méth)acrylique, de perfluoropolyéthers portant des groupes hydroxyle latéraux et / ou terminaux.
   De tels perfluoropolyéthers α,ω-diols sont décrits notamment dans EP-A-1057849 et sont commercialisés par la société AUSIMONT sous la dénomination FOMBLIN^{®} Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN^{®} des polyesters hyperramifiés. On trouvera sous la dénomination COMBURST^{®} de la société DENDRITECH des polyéthylèneamines hyperramifiées. Des poly(esteramide) hyperramifiés à extrémités hydroxyle sont commercialisés par la société DSM sous la dénomination HYBRANE^{®}.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

### 1a Composés réactifs additionnels

Selon un mode de réalisation, la composition conforme à l'invention peut comprendre en outre un composé réactif additionnel tel que :
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetramethyltetravinylsiloxane,
- des composés silazanes tels que l'hexaméthyldisilazane.

### 1b Catalyseur

La réaction d'hydrosilylation se fait avantageusement en présence d'un catalyseur qui peut être présent dans la composition conforme à l'invention, le catalyseur étant de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tétraméthyldisiloxane.

Le catalyseur peut être présent dans la composition conforme à la présente invention en une teneur allant 0,0001 % à 20 % en poids par rapport au poids total de la composition.

On peut également introduire dans la composition de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polyméthylvinylsiloxanes cycliques, et en particulier le tétravinyl tétraméthyl cyclotétrasiloxane, les alcools acétyléniques, de préférence volatiles, tels que le méthylisobutynol.

La présence de sels ioniques, tels que l'acétate de sodium, dans la composition peut avoir une influence dans la vitesse de polymérisation des composés.

De façon avantageuse, les composés X et Y sont choisis parmi les composés siliconés susceptibles de réagir par hydrosilylation ; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est le méthylhydrogénosiloxane.

A titre d'exemple d'une combinaison de composés X et Y réagissant par hydrosilylation, on peut citer les références suivantes proposée par la société Dow Corning : DC 7-9800 Soft Skin Adhesive Parts A & B, ainsi que les mélanges A et B suivants préparés par Dow Corning :

### MELANGE A :

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

### MELANGE B:

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

### 2/ Composés X et Y susceptibles de réagir par condensation

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle des fibres kératiniques, ou l'eau apportée par une source extérieure, par exemple par humidification préalable des fibres kératiniques (par exemple par un brumisateur).

Dans ce mode de réaction par condensation, les composés X et Y, identiques ou différents, peuvent donc être choisis parmi les composés siliconés dont la chaîne principale comprend au moins deux groupes alcoxysilane et / ou au moins deux groupes silanol (Si-OH), latéraux et / ou en bout de chaîne.

Selon un mode de réalisation avantageux, les composés X et / ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et / ou Y comprennent de préférence de façon majoritaire des unités de formule :

R⁹ₛSiO_{(4-s)/2}, (IV)

dans laquelle R⁹ représente indépendamment un radical choisi parmi les groupes alkyle comprenant de 1 à 6 atomes de carbone, le phényle, les groupes alkyle fluorés, et s est égal à 0, 1, 2 ou 3. De préférence, R⁹ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule :

(R⁹₂SiO₂)_{f}, (V)

dans laquelle R⁹ est tel que décrit ci-dessus, de préférence R⁹ est un radical méthyle, et
f est notamment tel que le polymère présente une viscosité à 25 °C allant de 0,5 à 3000 Pa.s, de préférence allant de 5 à 150 Pa.s et / ou
f est notamment un nombre allant de 2 à 5000, de préférence de 3 à 3000, mieux encore de 5 à 1000.

Ces composés X et Y polyorganosiloxanes comprennent au moins 2 groupes trialcoxysilanes terminaux par molécule de polymère, lesdits groupes ayant la formule suivante :

-ZSiR¹ₓ(OR)₃₋ₓ, (VI)

dans laquelle :
les radicaux R représentent indépendamment un groupe méthyle, éthyle, n-propyle, isopropyle, n-butyle, sec-butyle, isobutyle, de préférence un groupe méthyle ou éthyle,
R¹ est un groupe méthyle ou éthyle,
x est égal à 0 ou 1, de préférence x est égal à 0, et
Z est choisi parmi : les groupes hydrocarbonés divalents ne comportant pas d'insaturation éthylénique et comprenant de 1 à 18 atomes de carbone (groupes alkylène), les combinaisons de radicaux hydrocarbonés divalents et de segments siloxanes de formule (IX) suivante : 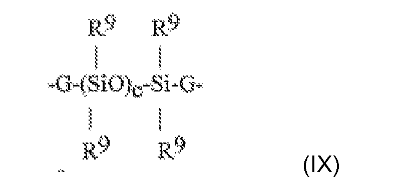
R⁹ étant tel que décrit plus haut, G est un radical hydrocarboné divalent ne comportant pas d'insaturation éthylénique et comprenant de 2 à 18 atomes de carbone et c est un entier allant de 1 à 6.
Z et G peuvent être notamment choisis parmi les groupements alkylène tels que le méthylène, l'éthylène, le propylène, le butylène, le pentylène, l'hexylène, les groupements arylène tels que le phénylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule CH₂=CH-SiR⁹₂- ou de formule R⁶₃- Si-, dans laquelle R⁹ est tel que défini plus haut et chaque groupe R⁶ est indépendamment choisi parmi les groupes R⁹ ou vinyle. On peut citer comme exemples de tels groupements terminaux les groupes triméthoxysilane, triéthoxysilane, vinyldiméthoxysilane et vinylméthyloxyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et / ou Y en particulier le polymère de formule : 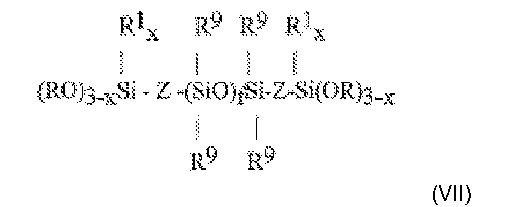 dans laquelle R, R¹, R⁹, Z, x et f sont tels que décrits plus haut.

Les composés X et / ou Y peuvent également comprendre un mélange de polymère de formule (VII) ci-dessus avec des polymères de formule (VIII) suivante : 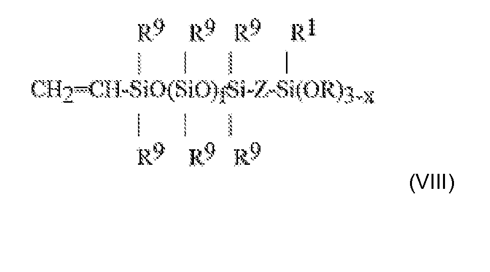 dans laquelle R, R¹, R⁹, Z, x et f sont tels que décrits plus haut.

Lorsque le composé X et / ou Y polyorganosiloxanes à groupe(s) alcoxysilane(s) comprend un tel mélange, les différents polyorganosiloxanes sont présents en des teneurs telles que les chaînes organosilyle terminales représentent moins de 40 %, de préférence moins de 25 % en nombre des chaînes terminales.

Les composés X et / ou Y polyorganosiloxanes particulièrement préférés sont ceux de formule (VII) décrits ci-dessus. De tels composés X et / ou Y sont décrits par exemple dans le document WO 01/96450.

Comme indiqué plus haut, les composés X et Y peuvent être identiques ou différents.

Selon une variante, l'un des 2 composés réactifs X ou Y est de nature silicone et l'autre est de nature organique. Par exemple le composé X est choisi parmi les oligomères ou polymères organiques ou les oligomères ou polymères hybrides organique / silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et / ou polyurées, polyéthers, polyoléfines, perfluoropolyéthers, dendrimères et polymères hyper-ramifiés organiques, et leurs mélanges.

Les polymères organiques de nature vinylique ou (méth)acrylique, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes, etc.

On peut citer par exemple les polymères (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et / ou polyurées, polyéthers, et porteurs de groupes alcoysilanes latéraux et / ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthylaminopropyltriméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux , on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et / ou Y polyorganosiloxanes, on peut citer les résines de type MO ou MT portant elle-même des extrémités alcoxysilanes et / ou silanols comme par exemple les résines poly(isobutylsilsesquioxane) fonctionnalisées par des groupes silanols proposées sous la référence SST-S7C41 (3 groupes Si-OH) par la société Gelest.

### 2a Composés réactifs additionnels

La composition conforme à la présente invention peut comprendre en outre un composé réactif additionnel comprenant au moins deux groupes alcoxysilane ou silanol.

On peut citer par exemple une ou des particules organiques ou minérales comprenant à leur surface des groupement alcoxysilanes et / ou silanols, par exemple des charges traitées en surface par de tels groupes.

### 2b Catalyseur

La réaction de condensation peut se faire en présence d'un catalyseur à base de métal qui peut être présent dans la composition conforme à l'invention. Le catalyseur utile dans ce type de réaction est de préférence un catalyseur à base de titane.

On peut citer notamment les catalyseurs à base de tétraalcoxytitane de formule :

Ti(OR²)_{y}(OR³)_{4-y},

dans laquelle R² est choisi parmi les radicaux alkyle tertiaires tels que le tert butyle, le tert amyle et le 2,4-diméthyl-3-pentyle ; R³ représente un radical alkyle comprenant de 1 à 6 atomes de carbone, de préférence un groupe méthyle éthyle, n-propyle, isopropyle, n-butyle, sec-butyle, hexyle et y est un nombre allant de 3 à 4, mieux de 3,4 à 4.

Le catalyseur peut être présent dans la composition conforme à la présente invention en une teneur allant 0,0001 % à 20 % en poids par rapport au poids total de la composition.

### 2c Diluant

La composition conforme à l'invention peut comprendre en outre une huile siliconée volatile (ou diluant) destinée à faire diminuer la viscosité de la composition. Cette huile peut être choisie parmi les silicones linéaires à chaîne courte telles que l'hexaméthyldisiloxane, l'octaméthyltrisiloxane, les silicones cycliques telles que l'octaméthylcyclotétrasiloxane, la décaméthylclopentasiloxane et leurs mélanges.

Cette huile siliconée peut représenter de 5 % à 95 %, de préférence de 10 à 80 % en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :

### Mélange A' :

| **Ingrédient (Nom INCI)** | **N°CAS** | **Teneurs (%)** | **Fonction** |
|---|---|---|---|
| Bis-Trimethoxysiloxyethyl Tetramethyldisiloxyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

### Mélange B' :

| **Ingrédient (Nom INCl)** | **N°CAS** | **Teneurs (%)** | **Fonction** |
|---|---|---|---|
| Disiloxane | 107-46-0 | 80-99 | Solvant |
| Tetra T Butyl Titanate | - | 1-20 | Catalyseur |

Il est d'ailleurs à noter que les composés X et Y identiques sont réunis dans le mélange A'.

### 3/ Réticulation en présence de peroxyde :

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux -CH₃ et / ou au moins deux chaînes latérales portant un groupement -CH₃.

Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux -CH₃ reliés à l'atome de silicium et / ou au moins deux chaînes latérales portant un groupement -CH₃. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation ou la réaction de condensation ou bien encore la réaction de réticulation en présence d'un peroxyde entre les composés X et Y est accélérée par un apport de chaleur en élevant par exemple la température du système entre 25 °C et 180 °C. Le système réagira notamment sur les fibres kératiniques.

D'une façon générale, quel que soit le type de réaction par laquelle les composés X et Y réagissent ensemble, le pourcentage molaire de X par rapport à l'ensemble des composés X et Y, c'est-à-dire le ratio X / (X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y / (X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,5 % à 95 % en poids par rapport au poids total de la composition, de préférence de 1 % à 90 % et mieux de 5 % à 80%.

Le composé Y peut représenter de 0,05 % à 95 % en poids par rapport au poids total de la composition, de préférence de 0,1 % à 90 % et mieux de 0,2 % à 80 %.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X / Y molaire allant de 0,05 à 20 et mieux de 0,1 à 10.

### Alcoxysilane comprenant un atome de silicium

Les alcoxysilanes utiles dans la présente invention peuvent être représentés par la formule générale R(₄₋ₙ)SiXₙ dans laquelle X représente un groupe hydrolysable tel que méthoxy, éthoxy, ou 2-méthoxy-éthoxy ; R est un radical organique monovalent comprenant de 1 à 12 atomes de carbone pouvant contenir des groupes tels que mercapto, epoxy, acrylyl, méthacrylyl, amino ou urée; et n est un nombre entier variant de 1 à 4, de préférence n est égal à 3,. A titre d'exemple d'alcoxysilanes utiles, on peut notamment citer le 3-mercaptopropyltriéthoxysilane ; les aminoalkyltrialcoxysilane tels que 3-aminopropyltriéthoxy silane tels que décrits dans la demande de brevet FR 2 789 896.

D'autres alcoxysilanes utiles sont par exemple cités dans la demande de brevet EP 1 216 022 qui décrit des alcoxysilanes comprenant au moins une chaîne hydrocarbonée comportant une fonction chimique solubilisante non basique. A ce titre, on peut citer le N-[(3-triméthoxysilyl)propyl-éthylènediaminetriacétate de sodium neutralisé par HCl fourni par la société GELEST.

Selon une variante, les alcoxysilanes utiles comprennent au moins une chaîne hydrocarbonée comprenant des atomes de fluor. A titre d'exemple, on peut citer les composés 3,3,3 trifluoropropyltriéthoxysilane ou le tridecafluorooctyltriéthoxysilane décrits dans la demande EP 1 510 197.

Selon un autre mode de réalisation, les alcoxysilanes utiles peuvent être des alcoxysilanes porteurs d'un groupement à fonction cosmétique tel que des colorants nitro aromatiques, anthraquinoniques, naphtoquinoniques, benzoquinoniques, azoiques, xanthéniques, triarylméthaniques, aziniques, indoaniliniques indophénoliques ou indoaminiques ; des groupes à effet réducteur tels que des groupes thiols, acide sulfinique ou sel d'acide sulfinique, ces alkoxysilanes pouvant être porteur d'un groupe non hydrolysable solubilisant tel que les groupes aminés, acides carboxyliques, acides sulfoniques, sulfates, ammoniums quaternaires, polyalcools, polyéther, et phosphates. A titre d'exemple, on peut citer l'aminopropyl-N-(4,2-dinitrophényl)amino propyl diéthoxysilane. De tels composés sont par exemple décrits dans la demande de brevet EP 1 216 023.

Les alcoxysilanes de l'invention peuvent être des alcoxysilanes aryl aminés. A titre d'exemple, on peut citer les composés suivants :
- le 3-(m-aminophénoxy)propyltriméthoxysilane, de formule : 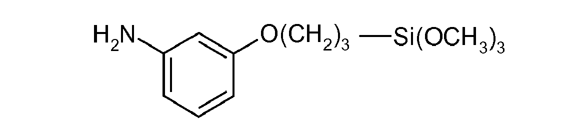 proposé par la société GELEST,
- le p-aminophényltriméthoxysilane, de formule : 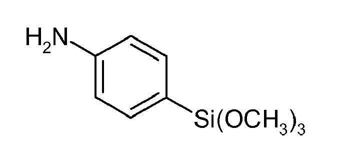 proposé par la société GELEST,
- le N-(2-aminoéthylaminométhyl)phénéthyltriméthoxysilane, de formule : 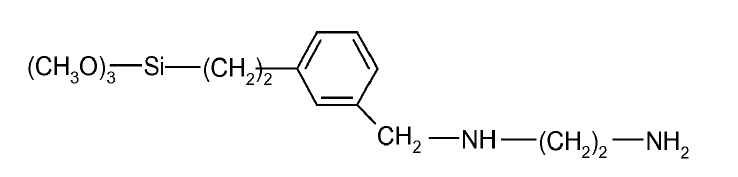 proposé par la société GELEST,

Un composé organique du silicium particulièrement préféré est le N-(2-aminoéthylaminométhyl)phénéthyltriméthoxysilane.
Les alcoxysilanes de l'invention peuvent être aussi des silanes à fonction aldéhyde ou acétal comme le triéthoxysilylbutyraldéhyde de formule : (CH₃CH₂O)₂Si(CH₂)₅CHO, le triéthoxysilylundecanol éthylèneglycol acétal (CH₃CH₂O)₃Si(CH₂)₁₀CH(OCH₂)₂ proposés par la société Gelest.
Les alcoxysilanes peuvent être aussi des silanes à amine non primaires comme la bis[3-(triéthoxysilyl)propyl]amine de formule (CH₃CH₂O)₃-Si(CH₂)₃NH(CH₂)₃SI(OCH₂CH₃)₃ proposé par la société Fluorochem, la bis [triméthoxysilylpropyl]amine de formule (CH₃O)₃-Si(CH₂)₃NH(CH₂)₃Si(OCH₃)₃ proposé par la société Gelest, la bis[méthyldiéthoxysilylpropyl]amine de formule (CH₃CH₂O)₂CH₃Si(CH₂)₃NH(CH₂)₃SiCH₃(OCH₂CH₃)₂ proposé par la société Gelest et la bis[3-triméthoxysilylpropyl]éthylènediamine de formule (CH₃O)₃Si(CH₂)₃NH(CH)₂NH(CH₂)₃Si(OCH₃)₃ proposé par la société Gelest.

Selon un mode de réalisation particulier, l'alcoxysilane est un trialcoxysilane comprenant un substituant aminé.

Le ou les composés organiques du silicium présents dans la composition selon l'invention peuvent représenter entre 0,1 et 50% en poids du poids total de la composition, de préférence de 1 à 25%.

La composition de l'invention peut contenir de l'eau ou un ou plusieurs solvants organiques, ou un mélange d'eau et d'un ou plusieurs solvants organiques.

Par solvant organique, on entend une substance organique liquide à la température de 25°C à pression atmosphérique (760mm de Hg) capable de dissoudre une autre substance sans la modifier chimiquement.

Le ou les solvants organiques utiles dans la présente invention sont distincts des composés X et Y définis précédemment.

Le solvant organique est par exemple choisi parmi les alcools aromatiques tels que l'alcool benzylique ; les alcools gras liquides , notamment en C10-C30; les polyols modifiés ou non tels que le glycérol, le glycol, le propylène glycol, le dipropylène glycol, le butylène glycol, le butyle diglycol ; les silicones volatiles telles que les silicones linéaires à chaîne courte l'héxaméthyldisiloxane, l'octométhyltrisiloxane, les silicones cycliques, tels que l'octaméthyl cyclotetrasiloxane, la décaméthylcyclopentasiloxane, la dodecaméthylcyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C5 à C10 ; les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C5-C10, l'acétone, la méthyléthylcétone, les esters d'acides en C1-C20 liquides et d'alcools en C1-C8 tels que l'acétate de méthyle, l'acétate de butyle, l'acétate d'éthyle et le myristate d'isopropyle, le diméthoxyéthane, le diéthoxyéthane, les alcools gras liquides en C10-C30 tels que l'alcool oléique, les esters d'alcools gras ou d'acide gras tels que les benzoates d'alcool gras en C10-C30 et leurs mélanges ; l'isononanoate d'isononyle, le malate d'isostéaryle, le tétra-isostéarate de pentaérythrityle, le trimélate de tridécyle ; l'huile de polybutène ; le mélange cyclopentasiloxane (14,7% en poids)/polydiméthylsiloxane dihydroxylé en positions α et γ (85,3% en poids), ou leurs mélanges.

Selon un mode de réalisation préféré, le solvant organique est constitué par une silicone ou un mélange de silicone tels que les polydiméthylsiloxanes liquides et les polydiméthylsiloxanes modifiées liquides, la viscosité de la silicone et/ou du mélange de silicone à 25°C est comprise entre 0.1cst et 1 000 000cst et plus préférentiellement entre 1 cst et 30 000cst.

On citera de préférence les huiles et mélanges d'huiles suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid,
- le mélange de polydiméthylsiloxane alpha-omega-dihydroxylé/ polydiméthylsiloxane commercialisé par Dow Corning sous le nom de DC 1503 Fluid,
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
et les mélanges respectifs de ces huiles.

Ces solvants organiques peuvent servir de diluant pour les réactions de polycondensation.

Le ou les solvants organiques et l'eau lorsqu'elle est présente représente généralement de 0,01 à 99 %, de préférence de 50 à 99 % en poids par rapport au poids total de la composition.

La composition de l'invention peut contenir, outre le ou les solvants organiques, de l'eau dans des proportions vatriant de 1 à 99 %, de préférence de 1 à 50 % par rapport au poids total de la composition. Cependant, selon un mode de réalisation particulier, la composition de l'invention est anhydre c'est-à-dire contenant moins de 1% en poids d'eau par rapport au poids total de la composition.

La composition de l'invention peut aussi se présenter sous forme d'une émulsion et/ou être encapsulée. Lorsque la composition est une émulsion, elle est par exemple constituée par une phase dispersée ou continue qui peut être de l'eau, des alcools aliphatiques en C1-C4 ou leurs mélanges et une phase organique insoluble dans l'eau.

La composition peut aussi contenir des charges qui sont généralement des composés substantiellement non colorés solides à température ambiante et pression atmosphérique, et insoluble dans la composition, même lorsque ces ingrédients sont portés à une température supérieure de la température ambiante.

A titre d'exemple, ces charges peuvent utiliser du talc ; du mica naturel ou synthétique, du kaolin, du carbonate de calcium colloïdal qui peut être traité ou non par de l'acide stéarique ou du stéarate, du carbonate et de l'hydro-carbonate de magnésium ; de l'hydroxyapatite, de la silice telle que les silices pyrogénées les silices précipitées, les silices traitées pour les rendre hydrophobes, du quartz moulu, de l'alumine, de l'hydroxyde d'aluminium, du dioxyde de titane, de la terre de diatomée, de l'oxyde de fer, du noir de carbone, et du graphite. Les silices synthétiques dont la surface est modifiée par des composés siliconés pour les rendre hydrophobe en surface sont particulièrement préférées. Ces charges se différentient les unes des autres par leurs propriétés de surface, les composés du silicone utilisés pour traiter la silice, et la façon dont le traitement en surface est réalisé. De telles charges permettent de réduire la viscosité de la formulation obtenue à partir des composés X et / ou Y. Par ailleurs, des charges renforçantes à base de résine peuvent aussi être utilisées. On préfère à titre de charge la silice, le carbonate de calcium, les charges à base de résine. A titre d'exemple, on peut citer les charges traitées Cab-O-Sil@TS-530, Aerosil@R8200 et Wacker HDX H2000.

La composition conforme à l'invention peut également contenir outre les composés X et Y, le ou les alcoxysilanes comprenant un seul atome de silicium et éventuellement le ou les solvants, au moins un agent utilisé habituellement en cosmétique choisi, par exemple, parmi des agents réducteurs, des corps gras, des plastifiants, des adoucissants, des agents anti-mousse, des agents hydratants, des pigments, des argiles, des filtres UV, des colloïdes minéraux, des peptisants, des parfums, des conservateurs, des tensio-actifs anioniques, cationiques, non ioniques ou amphotères, des polymères fixants ou non, des protéines, des vitamines, des colorants directs, des colorants d'oxydation, des agents nacrants, des propulseurs, et des épaississants minéraux ou organiques tels que le benzylidène-sorbitol et les N-acylaminoacides, les cires oxyéthylénées ou non, les paraffines, les acides gras solides en C₁₀-C₃₀ tels que l'acide stéarique, l'acide laurique, les amides gras ou d'acides gras solides.

Les compositions peuvent se présenter sous différentes formes galéniques tels qu'une lotion, une mousse aérosol, un après shampooing ou un shampooing, un gel, une cire. Les compositions peuvent être contenues dans un flacon pompe, un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges. On pourra le cas échéant utiliser des aérosols à poche(s) pouvant contenir une ou plusieurs poches.

Le procédé selon l'invention consiste à appliquer sur les fibres kératiniques :
- au moins un composé X et au moins un composé Y, l'un au moins des composés X et Y étant un composé siliconé, lesdits composés X et Y étant susceptibles de réagir ensemble par une réaction d'hydrosilylation, de condensation ou, de réticulation en présence de peroxyde lorsqu'ils sont mis en contact l'un avec l'autre ; et
- au moins un alcoxysilane comprenant un seul atome de silicium ; le ou les composés X, le ou les composés Y, le ou les un alcoxysilane comprenant un seul atome de silicium étant tels que définis précédemment.

Le ou les composés X, le ou les composés Y, le ou les alcoxysilanes comprenant un seul atome de silicium peuvent être appliqués sur les fibres kératiniques à partir de plusieurs compositions contenant le ou les composés X, le ou les composés Y, le ou les alcoxysilanes comprenant un seul atome de silicium, seuls ou en mélange, ou à partir d'une seule composition contenant le ou les composés X, le ou les composés Y, le ou les alcoxysilanes comprenant un seul atome de silicium.

Selon un mode de réalisation particulier de l'invention, on applique sur les fibres kératiniques une composition (A) comprenant le ou les composés X, le ou les composés Y, le ou les alcoxysilanes comprenant un seul atome de silicium.

Selon un autre mode de réalisation particulier de l'invention, on applique sur les fibres kératiniques une composition (B) comprenant le ou les alcoxysilanes comprenant un seul atome de silicium, et une composition (C) comprenant le ou les composés X et le ou les composés Y, l'ordre d'application des compositions (B) et (C) étant indifférent.

Selon un autre mode de réalisation particulier de l'invention, on applique sur les fibres kératiniques une composition (B) comprenant le ou les alcoxysilanes comprenant un seul atome de silicium, une composition (D) comprenant le ou les composés X et une composition (E) comprenant le ou les composés Y, l'ordre d'application des compositions (B), (D) et (E) étant indifférent.

Selon un autre mode de réalisation particulier de l'invention, on applique sur les fibres kératiniques une composition (F) comprenant le ou les composés X et le ou les alcoxysilanes comprenant un seul atome de silicium et une composition (E) comprenant le ou les composés Y, l'ordre d'application des compositions (F) et (E) étant indifférent.

Selon un autre mode de réalisation particulier de l'invention, on applique sur les fibres kératiniques une composition (D) comprenant le ou les composés X et une composition (G) comprenant le ou les composés Y et le ou les alcoxysilanes comprenant un seul atome de silicium, l'ordre d'application des compositions (D) et (G) étant indifférent.

Selon tous ces modes de réalisations, les compositions décrites ci-dessus peuvent contenir un ou plusieurs solvants organiques tels que décrits précédemment.

Selon un mode de réalisation préféré de l'invention, la composition comprenant le ou les alcoxysilanes comprenant un seul atome de silicium est appliquée avant la ou les compositions comprenant le ou les composés X et / ou le ou les composés Y.

Lorsque les composés X et Y sont susceptibles de réagir ensemble par une réaction de réticulation, on applique sur les fibres kératiniques au moins un peroxyde tel que défini précédemment.

Le ou les peroxydes peuvent être présents dans l'une ou l'autre ou dans plusieurs des compositions appliquées sur les fibres kératiniques citées ci-dessus ou dans une composition supplémentaire, auquel cas l'ordre d'application des différentes compositions sur les fibres kératiniques est indifférent.

Selon un mode de réalisation particulier de l'invention, on applique sur les fibres kératiniques au moins un catalyseur tel que défini précédemment pour activer la réaction entre le ou les composés X et le ou les composés Y.

Par exemple, le ou les catalyseurs peuvent être présents dans l'une ou l'autre ou dans plusieurs des compositions appliquées sur les fibres kératiniques citées ci-dessus ou dans une composition supplémentaire, auquel cas l'ordre d'application des différentes compositions sur les fibres kératiniques est indifférent.

Les catalyseurs qui sont avantageusement choisis sont ceux qui sont décrits ci-dessus.

Lorsque l'on applique sur les fibres kératiniques au moins un catalyseur et / ou au moins un peroxyde, le ou les composés X, le ou les composés Y, le ou les catalyseurs et/ou le ou les peroxydes ne sont pas stockés simultanément dans la même composition. Ils peuvent par contre être mélangés au moment de l'emploi.

Selon un autre mode de réalisation particulier de l'invention, on applique sur les fibres kératiniques au moins un composé réactif additionnel tel que défini précédemment.

Par exemple, le ou les composés réactifs additionnels peuvent être présents dans l'une ou l'autre ou dans plusieurs des compositions appliquées sur les fibres kératiniques citées ci-dessus ou dans une composition supplémentaire, auquel cas l'ordre d'application des différentes compositions sur les fibres kératiniques est indifférent.

Les différentes compositions mises en oeuvre dans le procédé conforme à l'invention peuvent être appliquées sur des cheveux secs ou humides.

Un séchage intermédiaire et/ou un rinçage peut être réalisé entre chaque application.

Chaque composition utile dans le procédé conforme à l'invention peut contenir en outre divers additifs cosmétiques conventionnels tels que définis précédemment.

Chaque composition utile dans le procédé conforme à l'invention comprend un milieu cosmétiquement acceptable, véhiculant le ou les composés X et / ou le ou les composés Y, et choisi de telle sorte que les composés X et Y soient aptes à réagir l'un avec l'autre par réaction d'hydrosilylation, de condensation ou, de réticulation en présence de peroxyde après l'application de la composition cosmétique sur les cheveux.

Le dépôt ainsi formé présente l'avantage d'avoir une faible solubilité attendue. En outre, il possède une bonne affinité pour la surface des fibres kératiniques, ce qui garantit une meilleure rémanence de l'ensemble du dépôt.

Lorsque les composés X et Y sont appliqués séparément, le dépôt en couches obtenu peut aussi être avantageux pour conserver les propriétés cosmétiques ou optiques du composé qui constitue la partie supérieure du dépôt.

Selon les mêmes procédés, il est possible de réaliser des superpositions multiples de couches de composés X et Y pour atteindre le type de dépôt souhaité (en termes de nature chimique, résistance mécanique, épaisseur, aspect, toucher...).

La présente invention a aussi pour objet un kit pour le traitement des fibres kératiniques comprenant au moins deux compositions conditionnées séparément, le kit comprenant :
- au moins un composé X et au moins un composé Y, l'un au moins des composés X et Y étant un composé siliconé, lesdits composés X et Y étant susceptibles de réagir ensemble par une réaction d'hydrosilylation, de condensation ou de réticulation en présence de peroxyde lorsqu'ils sont mis en contact l'un avec l'autre ;
- au moins un alcoxysilane comprenant un atome de silicium ; et éventuellement un ou plusieurs solvants organiques,
le ou les composés X, le ou les composés Y, le ou les alcoxysilanes comprenant un seul atome de silicium, le ou les solvants organiques étant tels que définis précédemment.

Selon un mode de réalisation particulier de l'invention, un premier compartiment contient la composition (B) telle que définie précédemment et un deuxième compartiment contient la composition (C) telle que définie précédemment.

Selon un autre mode de réalisation particulier, un premier compartiment contient la composition (B) telle que définie précédemment, un deuxième compartiment contient la composition (D) telle que définie précédemment et un troisième compartiment contient la composition (E) telle que définie précédemment.

Selon un autre mode de réalisation particulier de l'invention, un premier compartiment contient la composition (F) telle que définie précédemment et un deuxième compartiment contient la composition (E) telle que définie précédemment.

Selon un autre mode de réalisation particulier, un premier compartiment contient la composition (D) telle que définie précédemment et un deuxième compartiment contient la composition (G) telle que définie précédemment.

Lorsque les composés X et Y sont susceptibles de réagir ensemble par une réaction de réticulation, l'ensemble des compositions comprend de plus au moins un peroxyde tel que défini précédemment.

Par exemple, l'une ou l'autre ou plusieurs des compositions contenues dans le kit peut de plus comprendre au moins un peroxyde.

Le kit peut aussi contenir une composition supplémentaire comprenant, dans un milieu cosmétiquement acceptable, au moins un peroxyde.

Selon un mode de réalisation particulier de l'invention, l'ensemble des compositions comprend de plus au moins un catalyseur tel que défini précédemment, c'est-à-dire que l'une ou l'autre ou plusieurs des compositions contenues dans le kit peut de plus comprendre au moins un catalyseur ou que le kit comprend une composition supplémentaire contenant, dans un milieu cosmétiquement acceptable, au moins un catalyseur.

Lorsque l'ensemble des compositions comprend au moins un catalyseur et / ou au moins un peroxyde, le ou les composés X, le ou les composés Y, le ou les catalyseurs et / ou le ou les peroxydes ne sont pas stockés simultanément dans la même composition. Ils peuvent par contre être mélangés au moment de l'emploi.

La présente invention a également pour objet l'utilisation pour le traitement des fibres kératiniques de :
- au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- au moins un alcoxysilane comprenant un seul atome de silicium ; et éventuellement un ou plusieurs solvants organiques,
   le ou les composés X, le ou les composés Y, le ou les alcoxysilanes comprenant un seul atome de silicium, le ou les solvants organiques, étant tels que définis précédemment.

En particulier, la présente invention a pour objet l'utilisation pour le gainage rémanent des fibres kératiniques de :
- au moins un composé X et au moins un composé Y, l'un au moins des composés X et Y étant un composé siliconé, lesdits composés X et Y étant susceptibles de réagir ensemble par une réaction d'hydrosilylation, de condensation, ou de réticulation en présence de peroxyde lorsqu'ils sont mis en contact l'un avec l'autre ;
- au moins un alcoxysilane comprenant un seul atome de silicium ; et éventuellement un ou plusieurs solvants organiques,
   le ou les composés X, le ou les composés Y, le ou les alcoxysilanes comprenant un seul atome de silicium, le ou les solvants organiques étant tels que définis précédemment.

Les exemples non limitatifs suivants permettent d'illustrer l'invention sans en limiter sa portée.

### EXEMPLES

### EXEMPLE 1

Dans l'exemple suivants, on utilise les mélanges A' et B' suivants préparés par la société Dow Corning :

### Mélange A' :

| **Ingrédient (Nom INCl)** | **N°CAS** | **Teneurs (%)** | **Fonction** |
|---|---|---|---|
| Bis-Trimethoxysiloxyethyl Tetramethyldisiloxyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

### Mélange B' :

| **Ingrédient (Nom INCI)** | **N°CAS** | **Teneurs (%)** | **Fonction** |
|---|---|---|---|
| Disiloxane | 107-46-0 | 80-99 | Solvant |
| Tetra T Butyl Titanate | - | 1-20 | Catalyseur |

II est d'ailleurs à noter que les composés X et Y identiques sont réunis dans le mélange A'.

Les compositions suivantes sont réalisées

### Composition A

| | |
|---|---|
| Mélange A' | **50%** |
| **D5** | **40%** |
| Aminopropyltriéthoxysilane Z 6011 (Dow Corning) | 0,5% |
| Eau déminéralisée | 9,5% |

### Composition B

| | |
|---|---|
| Mélange B' | **50%** |
| **D5** | **50%** |

Les compositions A et B sont mélangées avec le rapport pondéral 10/1 avant l'application sur cheveux courts, secs. Les cheveux sont séchés à l'air libre pendant 30 minutes puis mis sous casque pendant 30 min. Nous obtenons des effets coiffants résistant à plusieurs shampooings. Le produit s'élimine en appliquant la D5 sur les cheveux traités. Les cheveux démaquillés ont un toucher particulièrement doux et cosmétique.

### EXEMPLE 2

Dans les exemples de compositions suivantes, on utilise la combinaison des mélanges A1 et B1 suivants préparés par la société Dow Corning :

### MELANGE A1 :

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

### MELANGE B1 :

| Ingrédient (Nom INCl) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

### Composition A

| | |
|---|---|
| Mélange A1 | 50% |
| D5 | 40% |
| Aminopropyltriéthoxysilane Z 6011 (Dow Corning) | 0,5% |
| Eau déminéralisée | 9,5% |

### Composition B

| | |
|---|---|
| Mélange B1 | 50% |
| D5 | 50% |

Les compositions A et B sont mélangées avec le rapport pondéral 1/1 avant l'application sur cheveux secs ou humides. Les cheveux sont séchés à l'air libre pendant 30 minutes puis mis sous casque pendant 30 min. Nous obtenons des effets coiffants résistant à plusieurs shampooings. Le produit s'élimine en appliquant la D5 sur les cheveux traités. Les cheveux démaquillés ont un toucher particulièrement doux et cosmétique.

## Revendications

1. Composition pour le traitement des fibres kératiniques comprenant :
- au moins un composé X et au moins un composé Y, l'un au moins des composés X et Y étant un composé siliconé, lesdits composés X et Y étant susceptibles de réagir ensemble par une réaction d'hydrosilylation en présence d'un catalyseur, de condensation, ou de réticulation en présence de peroxyde, lorsqu'ils sont mis en contact l'un avec l'autre ; et
- au moins un alcoxysilane comprenant un seul atome de silicium.

2. Composition selon la revendication 1 dans laquelle les composés X et Y sont susceptibles de réagir ensemble par hydrosilylation.

3. Composition selon la revendication 2 dans laquelle le composé X est choisi parmi les composés siliconés comprenant au moins deux groupements aliphatiques insaturés.

4. Composition selon la revendication 2 ou 3 dans laquelle le composé X est choisi parmi les polyorganosiloxanes comprenant au moins deux groupements aliphatiques insaturés.

5. Composition selon l'une des revendications 2 à 4 dans laquelle le composé X est choisi parmi les polyorganosiloxanes comprenant des unités siloxanes de formule : 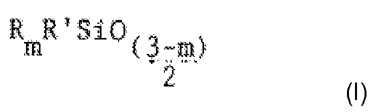 dans laquelle :
- R représente un groupe hydrocarboné monovalent, linéaire ou cyclique, comprenant de 1 à 30 atomes de carbone ;
- m est égal à 1 ou 2 ; et
- R' représente :
• un groupement hydrocarboné aliphatique insaturé comprenant de 2 à 10, ou
• un groupement hydrocarboné cyclique insaturé comprenant de 5 à 8 atomes de carbone.

6. Composition selon la revendication 5 dans laquelle R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone.

7. Composition selon la revendication 5 ou 6 dans laquelle les polyorganosiloxanes comprennent en outre des unités de formule : 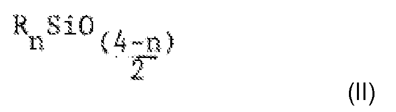 dans laquelle R est un groupe tel que défini dans la revendication 5, est n est égal à 1, 2 ou 3.

8. Composition selon la revendication 2 dans laquelle le composé X est choisi parmi les oligomères ou polymères organiques ou parmi les polymères ou oligomères hybrides organique / silicone, lesdits oligomères ou polymères portant au moins 2 groupements aliphatiques insaturés réactifs, le composé Y étant choisi parmi les hydrogénosiloxanes tels que définis précédemment.

9. Composition selon la revendication 8 dans laquelle le composé X est choisi parmi les polymères ou oligomères vinyliques, (méth)acryliques, les polyesters, les polyuréthanes et/ou les polyurées, les polyéthers, les perfluoropolyéthers, les polyoléfines, les dendrimères ou les polymères hyper-ramifiés organiques, lesdits polymères portant au moins 2 groupements aliphatiques insaturés réactifs et leurs mélanges.

10. Composition selon l'une des revendications 2 à 9 dans laquelle le composé Y est choisi parmi les polyorganosiloxanes comprenant au moins deux groupes Si-H libres.

11. Composition selon l'une des revendications 2 à 10 dans laquelle le composé Y est choisi parmi les polyorganosiloxanes comprenant des unités alkylhydrogènosiloxanes de formule suivante : 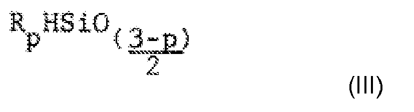 dans laquelle :
R représente un groupe hydrocarboné monovalent, linéaire ou cyclique, comprenant de 1 à 30 atomes de carbone, et p est égal à 1 ou 2.

12. Composition selon l'une quelconque des revendications précédentes dans laquelle les radicaux R représentent un groupement méthyle.

13. Composition selon l'une des revendications 2 à 12 dans laquelle les polyorganosiloxanes Y comprennent des groupes terminaux de formule CH₃SiO_{1/2}.

14. Composition selon l'une des revendications 2 à 13 comprenant au moins un catalyseur à base de platine ou d'étain.

15. Composition selon la revendication 14 dans laquelle le ou les catalyseurs représentent de 0,0001 % à 20 % en poids par rapport au poids total de la composition.

16. Composition selon la revendication 1 dans laquelle les composés X et Y sont susceptibles de réagir par condensation.

17. Composition selon la revendication 16 dans laquelle les composés X et Y, identiques ou différents, sont choisis parmi les composés siliconés dont la chaîne principale comprend au moins deux groupes alcoxysilane et / ou au moins deux groupes silanol (Si-OH), latéraux et / ou en bout de chaîne.

18. Composition selon la revendication 16 ou 17 dans laquelle les composés X et Y, identiques ou différents, sont choisis parmi les polyorganosiloxanes comprenant au moins deux groupes alcoxysilane.

19. Composition selon la revendication 17 ou 18 dans laquelle les polyorganosiloxanes comprennent de façon majoritaire des unités de formule :
R⁹ₛSiO_{(4-s)/2}, (IV)
dans laquelle les R⁹ représentent indépendamment un radical choisi parmi les groupes alkyles comprenant de 1 à 6 atomes de carbone, le phenyle, les groupes alkyl fluorés, et s est égal à 0, 1, 2 ou 3.

20. Composition selon la revendication 19 dans laquelle les polyorganosiloxanes comprennent des unités de formule :
(R⁹₂SiO₂)_{f}, (V)
dans laquelle R⁹ est tel que défini dans la revendication 19, et f est tel que le polymère présente une viscosité à 25 °C allant de 0,5 à 3000 Pa.s et f est un nombre allant de 2 à 5000.

21. Composition selon l'une des revendications 17 à 20 dans laquelle les polyorganosiloxanes comprennent au moins 2 groupes trialcoxysilane terminaux par molécule de polymère, lesdits groupes ayant la formule suivante :
-ZSiR¹ₓ(OR)₃₋ₓ, (VI)
dans laquelle :
les radicaux R représentent indépendamment un groupe méthyle, éthyle, n-propyle, isopropyle, n-butyle, sec-butyle, isobutyle ;
R¹ est un groupe méthyle ou éthyle ;
x est égal à 0 ou 1, de préférence x est égal à 0 ; et
Z est choisi parmi : les groupes hydrocarbonés divalents ne comportant pas d'insaturation éthylénique et comprenant de 1 à 18 atomes de carbone, les combinaisons de radicaux hydrocarbonés divalents et de segments siloxanes de formule : 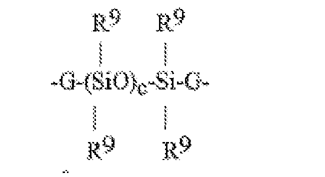
R⁹ étant tel que défini dans la revendication 19, G est un radical hydrocarboné divalent ne comportant pas d'insaturation éthylénique et comprenant de 2 à 18 atomes de carbone et c est un entier allant de 1 à 6.

22. Composition selon l'une des revendications 19 à 21 dans laquelle les polyorganosiloxanes sont choisis parmi les polymères de formule : 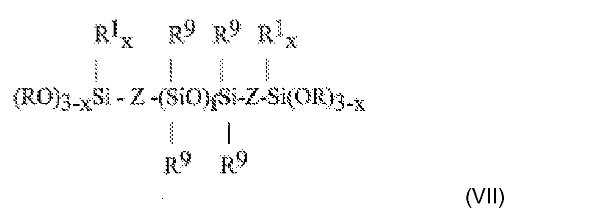 dans laquelle R, R¹, R⁹, Z, x et f sont tels que définis dans la revendication 21.

23. Composition selon l'une des revendications 17 à 22 comprenant au moins un catalyseur à base de titane.

24. Composition selon la revendication 23 dans laquelle le ou les catalyseurs sont présents en une teneur allant de 0,0001 % à 20 % en poids par rapport au poids total de la composition.

25. Composition selon la revendication 1 dans laquelle les composés X et Y sont susceptibles de réagir par réticulation en présence de peroxyde.

26. Composition selon l'une des revendications précédentes dans laquelle le composé X présente une masse moléculaire en poids (Mw) allant de 200 à 1 000 000.

27. Composition selon l'une des revendications précédentes dans laquelle le composé Y présente une masse moléculaire en poids (Mw) allant de 200 à 1 000 000.

28. Composition selon l'une quelconque des revendications précédentes dans laquelle le ou les alcoxysilanes sont représentés par la formule générale R(₄₋ₙ)SiXₙ dans laquelle X représente un groupe alcoxy ; R est un radical organique monovalent comprenant de 1 à 12 atomes de carbone pouvant contenir des groupes tels que mercapto, epoxy, acrylyl, méthacrylyl, fluoro, aryle, amino ou urée; et n est un nombre entier variant de 1 à 4.

29. Composition selon la revendication 28 dans laquelle l'alcoxysilane est choisi parmi le 3-aminopropyltriéthoxy silane, le 3-mercaptopropyltriéthoxysilane.

30. Composition selon l'une quelconque des revendications précédentes comprenant une ou plusieurs charges.

31. Composition selon l'une quelconque des revendications précédentes comprenant un solvant organique.

32. Composition selon l'une quelconque des revendications précédentes comprenant un solvant organique siliconé.

33. Procédé de traitement des fibres kératiniques **caractérisé par le fait que** l'on applique sur les fibres kératiniques la composition telle que définie à l'une quelconque des revendications 1 à 32.

34. Dispositif à plusieurs compartiments contenant au moins deux compositions telles que l'ensemble des compositions comprend :
- au moins un composé X et au moins un composé Y tels que définis à l'une quelconque des revendications 1 à 27 ;
- au moins un alcoxysilane comprenant un seul atome de silicium tel que défini à la revendication 1 et 28 à 30, et éventuellement
- un ou plusieurs solvants organiques.

35. Utilisation pour la coloration des fibres kératiniques de :
- au moins un composé X et au moins un composé Y tels que définis à l'une quelconque des revendications 1 à 27 ;
- au moins un alcoxysilane comprenant un seul atome de silicium tel que défini à l'une quelconque des revendications 1 et 28 à 30, et éventuellement un ou plusieurs solvants organiques.
